# FASCICULE DE BREVET EUROPEEN

(11) **EP 2 934 506 B1**
(45) Date de publication et mention de la délivrance du brevet: **17.01.2018**
(21) Numéro de dépôt: 13811513.4
(22) Date de dépôt: 19.12.2013
(51) Int. Cl.: A61K 31/221, C07C 229/24, C07C 227/18, C07C 229/08, A61K 9/00, A61P 17/00, A61P 17/06, A61P 17/08, A61P 17/10, A61P 29/00

(54) **DERIVES DE GLUTAMATE POUR LEUR UTILISATION TOPIQUE EN TANT QUE PRINCIPE ACTIF IMMUNOMODULATEUR**
GLUTAMATDERIVATE ZUR TOPISCHEN ANWENDUNG ALS IMMUNMODULIERENDER WIRKSTOFF
GLUTAMATE DERIVATIVES FOR TOPICAL USE AS IMMUNOMODULATORY ACTIVE INGREDIENT

(30) Priorité: 19.12.2012 FR 1262329
(43) Date de publication de la demande: 28.10.2015
(73) Titulaire: Pierre Fabre Dermo-Cosmétique, 92100 Boulogne-Billancourt (FR)
(72) Inventeur: REDOULES, Daniel, F-31300 Toulouse (FR); DAUNES-MARION, Sylvie, F-31000 Toulouse (FR); POIGNY, Stéphane, F-31600 Saubens (FR); ARIES, Marie-Françoise, F-31750 Escalquens (FR)
(74) Mandataire: Regimbeau
(86) Numéro de dépôt international: PCT/EP2013/077320
(87) Numéro de publication internationale: WO 2014/096155

(56) Documents cités:
- EP-A1- 0 572 167
- FR-A- 1 427 996
- US-A- 3 669 717
- DREW S. POCHÉ ET AL: "An Unconventional Method for Purifying the N-carboxyanhydride Derivatives of [gamma]-alkyl-L-glutamates", SYNTHETIC COMMUNICATIONS, vol. 29, no. 5, 1 mars 1999 (1999-03-01), pages 843-854, XP055080310, ISSN: 0039-7911, DOI: 10.1080/00397919908086042
- E KLIEGER ET AL: "VEREINFACHTE DARSTELLUNG UND REAKTIONEN VON CARBOBENZOXY-L-GLUTAMINSAURE-alpha-HALBEST ERN", LIEBIG'S ANNALEN, vol. 655, 1 janvier 1962 (1962-01-01), pages 195-210, XP055080255,
- HO V C ET AL: "SAFETY AND EFFICACY OF NONSTEROID PIMECROLIMUS CREAM 1% IN THE TREATMENT OF ATOPIC DERMATITIS IN INFANTS", JOURNAL OF PEDIATRICS, MOSBY-YEAR BOOK, ST. LOUIS, MO, US, vol. 142, no. 2, 1 février 2003 (2003-02-01), pages 155-162, XP009006977, ISSN: 0022-3476, DOI: 10.1067/MPD.2003.65

## Description

Le domaine de la présente invention concerne de nouveaux dérivés de glutamate et leur utilisation en tant que principe actif immunomodulateur.

Plus particulièrement, la présente invention se rapporte à de nouvelles compositions intéressantes pour leur utilisation topique dans le traitement et dans la prévention des dermatoses inflammatoires telles que la dermatite atopique, l'eczéma de contact, l'acné, la dermatite séborrhéique, la rosacée ou le psoriasis.

Les applications à ce jour décrites concernant les esters de l'acide glutamique visent entre autres des compositions cosmétiques destinées à favoriser la croissance capillaire (EP0572167). D'autres antériorités décrivent certains esters de l'acide glutamique en tant qu'intermédiaires de synthèse.

La dermatite atopique (DA) est une pathologie chronique inflammatoire qui affecte près de 20 % des enfants et dont la prévalence est en nette augmentation dans les pays développés. C'est une affection plurifactorielle, dont le phénotype est modulé par des facteurs relevant à la fois de facteurs génétiques et immunologiques mais aussi hormonaux et environnementaux, qui se combinent de façons diverses et s'expriment de manière très polymorphe chez les patients. Actuellement, on distingue la forme de la DA allergique IgE-dépendante, qui affecte environ 80 % des patients et la forme non allergique qui concerne une minorité de patients chez lesquels on retrouve une concentration sérique en IgE normale. En outre, les études épidémiologiques semblent indiquer que la DA débute chez l'enfant sous une forme non allergique et évolue ensuite vers la forme allergique (Novak N et al., Dichotomic nature of atopic dermatitis reflected by combined analysis of monocyte immunophenotyping and single nucleotide polymorphisms of the interleukin-4/interleukin-13 receptor gene: the dichotomy of extrinsic and intrinsic atopic dermatitis, J Invest Dermatol. 2002 Oct;119(4):870-5, 2002 ; Allam JP et al., Recent highlights in the pathophysiology of atopic eczema, Int Arch Allergy Immunol. 2005 Feb;136(2):191-7).

Sur le plan immunologique, le développement de la lésion inflammatoire intervient après une phase de sensibilisation avec des allergènes présents dans l'environnement soit protéiques (cas de la dermatite atopique) soit chimique (eczéma allergique de contact). Les lésions d'eczéma surviennent, après une nouvelle exposition à l'allergène et sont dues à une infiltration des cellules inflammatoires du sang vers la peau. Les sous-populations lymphocytaires T CD4⁺ activées lors de cette phase présentent un profil particulier de production de cytokines de type Th2 (IL-4, IL-5 et IL-13) et sont impliquées dans le recrutement des cellules effectrices de la réaction retardée et entraînent des diminutions de synthèse de filaggrine et des céramides lesquelles caractérisent les anomalies de la barrière cutanée observées chez les patients porteurs de la DA (Cytokine modulation of atopic dermatitis filaggrin skin expression, Howell MD et al., J Allergy Clin Immunol. 2007 Jul;120(1):150-5), (Imokawa et al., Decreased level of ceramides in stratum corneum of atopic dermatitis: an etiologic factor in atopicdry skin, J Invest Dermatol. 1991 Apr;96(4):523-6 1991). Dans ce contexte, l'altération de la barrière cutanée favorise la pénétration des allergènes et l'activation des lymphocytes T spécifiques. En outre, ces cytokines sont responsables de la synthèse d'IgE par les plasmocytes descendants de lymphocytes B activés par l'antigène. Il existe ainsi un cercle vicieux d'entretien de la réponse allergique. Les cellules présentatrices d'antigènes chargées en IgE présentent une plus grande quantité d'épitopes aux lymphocytes et favorisent une réponse IgE.

La production d'IL-4 caractérise la différenciation TH2, laquelle joue un rôle crucial dans le développement de la réponse IgE.

En outre, il a été montré que les lymphocytes TH17 produisent des cytokines proinflammatoires et jouent un rôle important dans la pathogénie de la DA. En effet, dans une étude pharmaco-clinique, les auteurs trouvent une augmentation du pourcentage des lymphocytes TH17 dans le sang et dans la peau de sujets porteurs de DA, et que cette augmentation est positivement et significativement corrélée avec la gravité de la DA (Koga C, Kabashima K, Kobayashi M, Tokura Y. Possible pathogenic role of TH17 cells for atopic dermatitis. J Investig Dermatol 2008;128:2625-30). Enfin, on peut signaler la prédominance de la réponse TH1 dans les lésions chroniques de la DA (von Bubnoff et al, Natural killer cells in atopic and autoimmune diseases of the skin, J Allergy Clin Immunol. 2010 Jan;125(1):60-8).

Les traitements classiques de la dermatite atopique utilisent notamment des dermocorticoïdes ou des immunosuppresseurs, molécules capables de supprimer la réponse immune lymphocytaire T.

Les efficacités cliniques d'immunosuppresseurs comme le tacrolimus ou le pimecrolimus ont été établies par de nombreuses études :
- De Prost et al., Double-blind randomized placebo-controlled trial of local cyclosporine in atopic dermatitis, Arch Dermatol. 1989 Apr;125(4):570 ;
- Ho VC et al., Safety and efficacy of nonsteroid pimecrolimus cream 1% in the treatment of atopic dermatitis in infants, J Pediatr. 2003 Feb;142(2):155-62 ;
- Reitamo et al., Topical noncorticosteroid immunomodulation in the treatment of atopic dermatitis, Am J Clin Dermatol. 2002;3(6):381-8. Review.

Cependant les dermocorticoïdes ou les immunosuppresseurs ne sont pas dénués d'effets indésirables notamment chez l'enfant.

La DA se caractérise par des éruptions répétées pendant plusieurs années. Elle évolue par poussées entrecoupées de rémissions spontanées.

La maladie doit être traitée au long cours. Il existe ainsi un besoin et une forte demande pour mettre en évidence des alternatives thérapeutiques à ces dermatoses inflammatoires. L'utilisation d'immunomodulateurs par voie locale, fait aujourd'hui partie de l'arsenal thérapeutique pour traiter la dermatite atopique ainsi que d'autres dermatoses comme le psoriasis ou l'eczéma de contact.

Menant des recherches actives sur ces dermatoses inflammatoires, la Demanderesse a constaté de manière particulièrement surprenante que des dérivés de L-glutamate de formule générale I possédaient des propriétés à la fois de modulation de la réponse immune et modulation de la réponse inflammatoire.

Dans le cadre de cette invention, il a donc été envisagé le développement de molécules dérivées de L-glutamate (monoester en position 5 de l'acide L-glutamique), conçues pour des applications topiques, et capables de moduler l'activation des populations lymphocytaires et kératinocytaires.

Au sens de la présente invention, on entend par « dérivés de L-glutamate » les composés qui répondent à la formule générale I suivante : 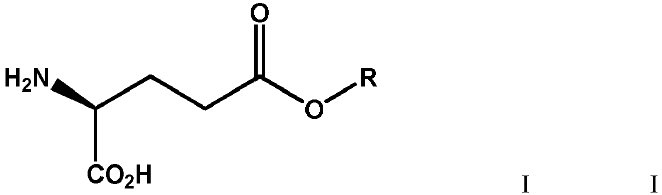 dans laquelle R représente un groupe alkyle, linéaire ou ramifié, en C₅ à C₂₅ et de préférence de C₅ à C₁₄ pour leur utilisation topique en tant que principe actif dermatologique destiné au traitement et/ou à la prévention des maladies inflammatoires de la peau.

Par "radical alkyle", on entend au sens de la présente invention une chaîne hydrocarbonée aliphatique linéaire ou ramifiée saturée et comprenant le nombre d'atomes de carbone spécifié.

Selon un autre aspect de l'invention, les utilisations et compositions dermatologiques destinées au traitement et/ou à la prévention des dermatoses inflammatoires, concernent également les dérivés de glutamate non pas uniquement sous forme d'énantiomères L-pur, mais également sous forme de tous mélanges des deux énantiomères D et L avec toutefois de préférence une fraction prépondérante d'énantiomère L.

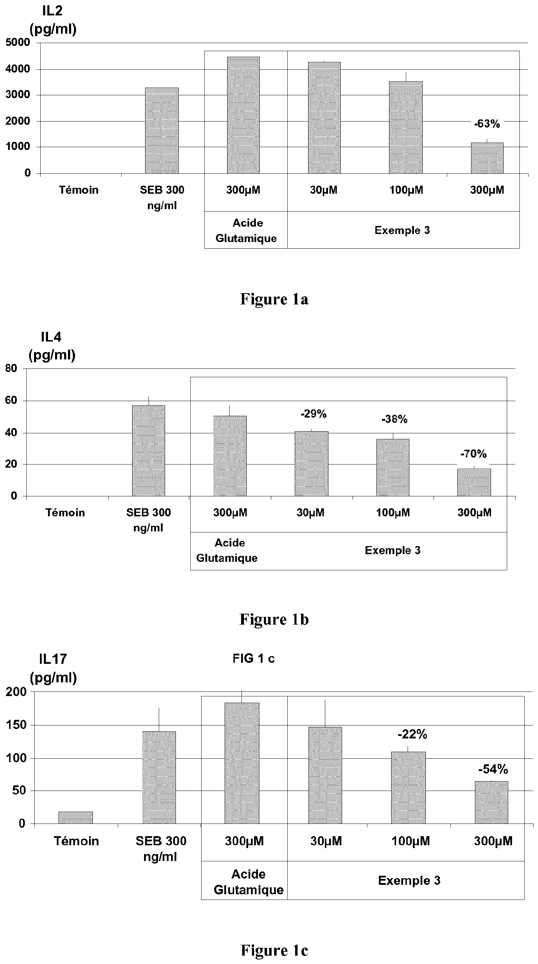

Les composés selon l'invention présentent des efficacités immunomodulatrices supérieures à celles obtenues avec l'acide glutamique seul vis-à-vis des réponses lymphocytaires TH1, TH2 et TH17 (figures 1a, 1b et 1c respectivement obtenues avec le composé de l'exemple 3). Ces dérivés présentent donc un intérêt potentiel en tant qu'immunorégulateur chez des patients atteints de maladies inflammatoires cutanées telles que la dermatite atopique, l'eczéma de contact, ou le psoriasis.

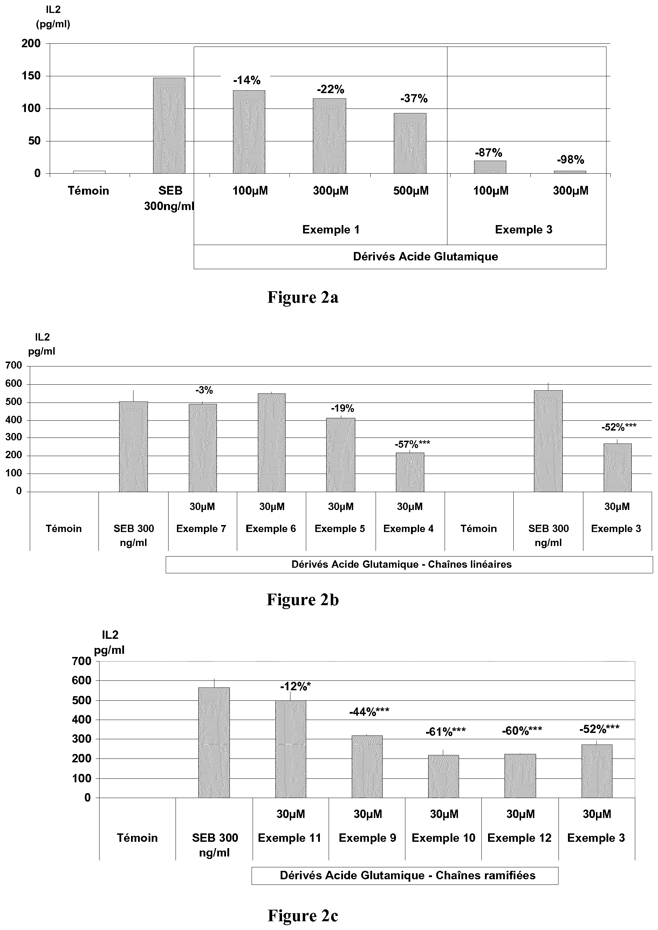

Dans un mode de réalisation particulier de l'invention, l'activité des dérivés de formule générale I a été évaluée en fonction de leur longueur de chaîne. L'activité est optimale pour les composés dont les longueurs de chaînes sont comprises entre C₅ et C₁₄ (figure 2a et 2b). Selon une autre mode d'exécution de l'invention, la présence d'une chaîne ramifiée courte permet de maintenir l'activité immunomodulatrice comparativement au composé selon l'exemple 3 (figure 2c).

Dans un mode de réalisation préférée de l'invention, les composés de formule I sont les nouveaux composés :
- 5 (3,7-diméthyloctyl)-Lglutamate ou acide 2-amino-5(3,7-diméthyloctyloxy)-5-oxopentanoïque
- 5 (nonan-2-yl)-L-glutamate ou acide 2-amino-5(nonan-2-yloxy)-5-oxopentanoïque
- 5 (nonan-5-yl)-L-glutamate ou acide 2-amino-5(nonan-5-yloxy)-5-oxopentanoïque
- 5 (2-hexyldecyl)-L-glutamate ou acide 2-amino-5(2-hexyldecyl oxy)-5-oxopentanoïque
- 5 (2-éthylhexyl)-L-glutamate ou acide 2-amino-5(2-éthylhexyl oxy)-5-oxopentanoïque

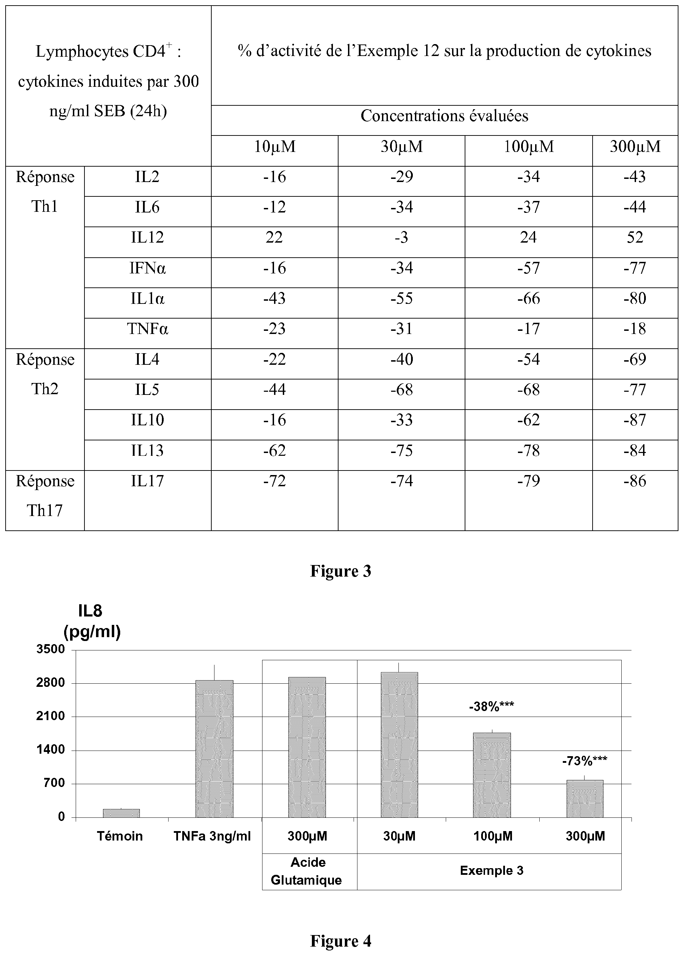

Par ailleurs, les composés de formule I selon l'invention manifestent également une activité anti-inflammatoire qui n'est pas retrouvée avec l'acide glutamique (figure 4 pour le composé selon l'exemple 3). Cette activité supplémentaire de ces dérivés élargie leur champ d'applications thérapeutiques à d'autres dermatoses inflammatoires telles que l'acné, la rosacée ainsi que la dermite séborrhéique.

Les dérivés selon l'invention présentent donc un intérêt en tant qu'agent anti-inflammatoire et immunorégulateur chez des patients atteints de dermatoses inflammatoires telles que la dermatite atopique, l'eczéma de contact, l'acné, la dermite séborrhéique, la rosacée ou le psoriasis.

Selon un mode de réalisation particulier de l'invention, les composés de formule générale (I) peuvent être choisis parmi la liste des composés suivants :
- 5-(n-pentyl)-L-glutamate ou acide 2-amino-5-oxo-5-(pentyloxy)pentanoïque
- 5-(n-hexyl)-L-glutamate ou acide 2-amino-5-(hexyloxy)-5-oxopentanoïque
- 5-(n-nonyl)-L-glutamate ou acide 2-amino-5-(nonyloxy)-5-oxopentanoïque
- 5-(n-dodécyl)-L-glutamate ou acide 2-amino-5-(dodécyloxy)-5-oxopentanoïque
- 5-(n-tétradécyl)-L-glutamate ou acide 2-amino-5-oxo-5(tétradécyl)pentanoïque
- 5-(n-hexadecyl)-L-glutamate ou acide 2-amino-5-(hexadecyloxy)-5-oxopentanoïque
- 5-(n-octadécyl)-L-glutamate ou acide 2-amino-5-(octadécyloxy)-5-oxopentanoïque
- 5-(3,7-diméthyloctyl)-L-glutamate ou acide 2-amino-5(3,7-dimethyloctyloxy)-5-oxopentanoïque
- 5-(nonan-2-yl)-L-glutamate ou acide 2-amino-5(nonan-2-yloxy)-5-oxopentanoïque
- 5-(nonan-5-yl)-L-glutamate ou acide 2-amino-5(nonan-5-yloxy)-5-oxopentanoïque
- 5-(2-hexyldecyl)-L-glutamate ou acide 2-amino-5(2-hexyldecyl oxy)-5-oxopentanoïque
- 5-(2-éthylhexyl)-L-glutamate ou acide 2-amino-5(2-éthylhexyl oxy)-5-oxopentanoïque

La présente invention concerne également l'utilisation topique des compositions dermatologiques ou cosmétiques comprenant un composé de formule générale (I) dans le traitement et/ou à la prévention des dermatoses inflammatoires.

Selon une autre caractéristique de l'invention, l'actif immunomodulateur se présente sous la forme d'une composition dermatologique ou cosmétique contenant au moins un composé de formules I à raison de 0,01 % à 20 %, de préférence de 0,1 % à 10 % en poids, et plus particulièrement de 1% à 5% en poids par rapport au poids total de la composition.

La composition dermatologique ou cosmétique selon l'invention comprend, en outre, un ou plusieurs excipients usuels dermatologiquement ou cosmétiquement compatibles.

Les excipients dermatologiquement ou cosmétiquement compatibles peuvent être tout excipient parmi ceux connus de l'homme de l'art en vue d'obtenir une composition pour l'application topique sous forme d'un lait, d'une crème, d'un baume, d'une huile, d'une lotion, d'un gel, d'un gel moussant, d'une pommade, d'un spray, etc.

L'invention concerne également un procédé de préparation des composés de formule I, caractérisé en ce qu'on fait réagir le N-alpha-benzylcarbonyl-L-glutamic-acid-alpha-benzyl ester avec un alcool de formule R-OH où R à la signification donnée précédemment et le produit obtenu est déprotégé par hydrogénation catalytique.

L'invention sera mieux comprise à la lecture des résultats ci-dessous qui l'illustrent.

### LEGENDE DES FIGURES :

- figures 1a, 1b et 1c : Mise en évidence de l'activité immuno-régulatrice du composé selon l'exemple 3 vis-à-vis des sécrétions d'IL-2 (1a), d'IL-4 (1b) et d'IL-17 (1c) par les lymphocytes humains.
- figures 2a, 2b et 2c : Mise en évidence de l'activité immuno-régulatrice des dérivés alkylés à chaîne courte linéaire (2a), à chaîne longue linéaire (2b) et à chaîne courte ramifiée (2c) vis-à-vis de la sécrétion d'IL-2 par les lymphocytes humains.
- figure 3 : Mise en évidence de l'activité immuno-régulatrice du composé selon l'exemple 12 vis-à-vis des sécrétions de cytokines impliquées dans les réponses immunitaires de types Th1 Th2 et Th17.
- figure 4 : Mise en évidence de l'activité anti-inflammatoire du composé selon l'exemple 3 vis-à-vis de la production d'IL-8 par les kératinocytes humains.

### A/ EVALUATION PHARMACOLOGIQUE :

A.1 - Mise en évidence de l'activité immuno-régulatrice : Etude de la sécrétion d'IL2, d'IL-4 et d'IL-17 pour respectivement les réponses lymphocytaires TH1, TH2 et TH17.

Les activités pharmacologiques des esters selon l'invention ont été mises en évidence comme suit :
- Les cellules humaines mononucléées sanguines (Peripheral Blood Mononuclear Cells, PBMC) sont isolées à partir de poches Buffy-Coat de l'Etablissement Français du Sang (EFS) Pyrénées Méditerranée, sur gradient de Ficoll (Milieu de Séparation des Lymphocytes, densité 1.077g/ml) ; les lymphocytes CD4⁺ sont purifiés à partir de cette population cellulaire par immunosélection magnétique (CD4⁺ T Cell Isolation Kit II, Réf 130-091-155 Miltenyi Biotec) puis distribués en plaques 24 puits dans du milieu de culture RPMI 5% SVF ; ils sont alors pré-incubés avec les principes actifs à évaluer pendant 1 heure puis stimulés pendant 24 heures par 300 ng/ml SEB (Entérotoxine B de Staphylococcus aureus). Les dosages de cytokines lymphocytaires sont réalisés à partir d'une prise d'essai du surnageant selon les méthodes suivantes :
   - IL2 (TH1), IL4 (TH2), sont évaluées en cytométrie en flux par CBA (Réf 550749, BECTON Dickinson),
   - IL17 (TH17) est évaluée par ELISA, Human IL17 Quantikine (Réf D1700, R&D Systems),
   - IL2 (TH1) est également évaluée par ELISA, Duoset IL2 (Réf DY202, R&D Systems).

La modulation de la réponse immune observée est illustrée par les dessins annexés sur lesquels :
Figures 1 : Mise en évidence de l'activité immuno-régulatrice du composé selon l'exemple 3 vs l'acide glutamique vis-à-vis des productions d'IL-2 (1a), d'IL-4 (1b) et d'IL-17 (1c) par les lymphocytes humains ; cette activité est dose-dépendante et atteint 63%, 70% et 54% d'inhibition des productions d'IL-2, IL-4 et IL-17 respectivement à la concentration 300µM.
Figure 2 : Mise en évidence de l'activité immuno-régulatrice des dérivés alkylés à chaîne courte linéaire (2a), à chaîne longue linéaire (2b) et à chaîne courte ramifiée (2c) vis-à-vis de la sécrétion d'IL-2 par les lymphocytes humains ; l'activité est optimale pour les composés dont les longueurs de chaînes sont comprises entre C₅ et C₁₄ et la présence d'une chaîne ramifiée courte permet de maintenir cette activité comparativement au composé selon l'exemple 3.
Figure 3 : Mise en évidence de l'activité immuno-régulatrice du composé selon l'exemple 12 vis à vis des productions des cytokines Th1, Th2, Th17 par les lymphocytes humains ; cette activité est dose-dépendante et atteint 43%, 44%, 77%, 80%, 18% d'inhibition des productions d'IL2, IL6, IFNγ, IL1β, TNFα, 69%, 77%, 87%, 84% d'inhibition des productions d'IL4, IL5, IL10, IL13, et 86% d'inhibition de la production d'IL17 à la concentration 300µM.

La présence d'un caractère lipophile sur le motif glutamate permet de moduler l'activation lymphocytaire des TH2, des TH17 et des TH1 induite par l'entérotoxine B (SEB), super-antigène du staphylocoque doré.
A.2 - Mise en évidence de l'activité anti-inflammatoire : Etude de la sécrétion d'IL8 pour la réponse kératinocytaire.
   - Les kératinocytes HaCaT distribués en plaques 24 puits sont pré-incubés pendant 2 heures avec les principes actifs à évaluer puis stimulés pendant 24 heures par 10ng/ml TNFα. Le dosage d'interleukine 8 est réalisé à partir d'une prise d'essai du surnageant selon la méthode suivante :
      - IL8 est évaluée par ELISA, Duoset IL8 (Réf DY208E, R&D Systems).

La modulation de la réponse inflammatoire observée est illustrée par le dessin annexé sur lequel :
Figure 4 : Mise en évidence de l'activité anti-inflammatoire de l'exemple 3 vs l'acide glutamique vis-à-vis de la production d'IL-8.

### EXEMPLES DE SYNTHESE DES COMPOSES SELON L'INVENTION

### Procédure 1

Cette procédure est adaptée de celles décrites dans le brevet FR 1 427 996, MERCK & CO, 1965.

### Exemple 1

### 5-(n-pentyl)-L-glutamate ou acide 2-amino-5-oxo-5-(pentyloxy)pentanoïque

L'acide L-glutamique (3g, 1eq) est mis en suspension dans un mélange de tert-butanol (10.5eq) et pentanol 99% (4eq). Après chauffage du milieu réactionnel à 40°C, H₂SO₄ 95% (1.5eq) est ajouté goutte à goutte. La température de chauffage est alors augmentée jusqu'à 65°C jusqu'à l'obtention d'une solution limpide. Finalement, la température est maintenue pendant vingt heures à 65°C. Après avoir arrêté le chauffage, la triéthylamine (0.57eq) est ajoutée goutte à goutte au mélange réactionnel aussi rapidement que possible. Sont ensuite ajoutés 5mL d'eau et 66mL d'éthanol. Enfin, à température ambiante, 2,1eq de triéthylamine sont ajoutés ce qui amorce la précipitation. Le milieu réactionnel est alors laissé sous agitation pendant 30 minutes. Le précipité est ensuite filtré puis séché pendant une heure sous vide à 50°C et lavé avec 13mL d'éthanol puis 13mL d'éther diéthylique. Le produit obtenu est séché sous vide à 30°C pendant une nuit puis recristallisé en ajoutant 40mL d'une solution isopropanol : eau / 1:1 et en chauffant à 83°C jusqu'à dissolution complète. Le mélange est ensuite ramené à température ambiante et le produit cristallise. Le précipité est alors lavé avec 4mL du mélange isopropanol : eau / 1 :1 puis avec 13mL d'éthanol et enfin 13mL d'éther diéthylique. Le produit obtenu est isolé sous forme de cristaux blancs.
Rendement : 10% 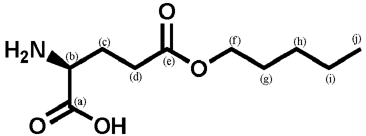
RMN (¹H, D₂O) : δ (ppm) : 0,9 (t, 3H, CH₃₍ⱼ₎) ; 1,3 (m, 4H, CH₂₍ᵢ₋ₕ₎) ; 1,7 (quint, 2H, CH_{2(g)}) ; 2,1 (m, 2H, CH_{2(c)}) ; 2,6 (m, 2H, CH_{2(d)}) ; 3,7 (t, 1H, CH_{(b)}) ; 4,1 (t, 2H, CH_{2(f)}).
RMN (¹³C, D₂O) : δ (ppm) : 16,1 (CH₃₍ⱼ₎) ; 24,5 (CH₂₍ᵢ₎) ; 28,4 (CH_{2(c)}) ; 30,2 (CH₂₍ₕ₎) ; 30,3 (CH_{2(g)}) ; 32,9 (CH_{2(d)}) ; 56,9 (CH_{(b)}) ; 68,9 (CH_{2(f)}) ; 174,5 (C₍ₑ₎) ; 177,9 (C₍ₐ₎).
SM : ESI+ : [M+H]⁺ : 218,2 (100%)

### Exemple 2

### 5(-n-hexyl)-L-glutamate ou acide 2-amino-5-(hexyloxy)-5-oxopentanoïque

L'acide L-glutamique (3g, 1eq) est mis en suspension dans un mélange de tert-butanol (10.5eq) et hexanol 99% (4eq). Après chauffage du milieu réactionnel à 40°C, H₂SO₄ 95% (1.5eq) est ajouté goutte à goutte. La température de chauffage est alors augmentée jusqu'à 65°C jusqu'à l'obtention d'une solution limpide. Finalement, la température est maintenue pendant une heure à 65°C. Après avoir arrêté le chauffage, la triéthylamine (0.57eq) est ajoutée goutte à goutte au mélange réactionnel aussi rapidement que possible. Sont ensuite ajoutés 5mL d'eau et 66mL d'éthanol. Enfin, à température ambiante, 2,1eq de triéthylamine sont ajoutés ce qui amorce la précipitation. Le milieu réactionnel est alors laissé sous agitation pendant 30minutes. Le précipité est ensuite filtré puis séché pendant une heure sous vide à 50°C et lavé avec 13mL d'éthanol puis 13mL d'éther diéthylique. Le produit obtenu est séché sous vide à 30°C pendant une nuit puis recristallisé en ajoutant 40mL d'une solution isopropanol:eau / 1:1 et en chauffant à 83°C jusqu'à dissolution complète. Le mélange est ensuite ramené à température ambiante et le produit cristallise. Le précipité est alors lavé avec 4mL du mélange isopropanol:eau / 1:1 puis avec 13mL d'éthanol et enfin 13mL d'éther diéthylique. Le produit obtenu est isolé sous forme de cristaux blancs.
Rendement : 17% 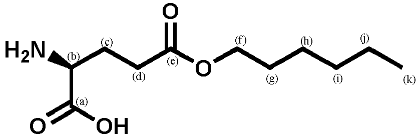
RMN (¹H, D₂O) : δ (ppm) : 0,88 (t, 3H, CH₃₍ₖ₎) ; 1,3 (m, 6H, CH₂₍ⱼ₋ₕ₎) ; 1,7 (quint, 2H, CH_{2(g)}) ; 2,1 (m, 2H, CH_{2(c)}) ; 2,6 (m, 2H, CH_{2(d)}) ; 3,7 (t, 1H, CH_{(b)}) ; 4,1 (t, 2H, CH_{2(f)}).
RMN (¹³C, D₂O) : δ (ppm) : 16,2 (CH₃₍ₖ₎) ; 24,8 (CH₂₍ⱼ₎) ; 27,7 (CH_{2(c)}) ; 28,4 (CH₂₍ₕ₎) ; 30,6 (CH_{2(g)}) ; 32,9 (CH_{2(d)}) ; 33,6 (CH₂₍ᵢ₎) ; 56,9 (CH_{(b)}) ; 68,9 (CH_{2(f)}) ; 174,5 (C₍ₑ₎) ; 177,9 (C₍ₐ₎).
SM : ESI+ : [M+H]⁺ : 232,1 (100%)

### Exemple 3

### 5-(n-nonyl)-L-glutamate ou acide 2-amino-5-(nonyloxy)-5-oxopentanoïque

L'acide L-glutamique (3g, 1eq) est mis en suspension dans un mélange de tert-butanol (10.5eq) et nonanol 99% (4eq). Après chauffage du milieu réactionnel à 40°C, H₂SO₄ 95% (1.5eq) est ajouté goutte à goutte. La température de chauffage est alors augmentée jusqu'à 65°C jusqu'à l'obtention d'une solution limpide. Finalement, la température est maintenue pendant quatre heures à 65°C. Après avoir arrêté le chauffage, la triéthylamine (0.57eq) est ajoutée goutte à goutte au mélange réactionnel aussi rapidement que possible. Sont ensuite ajoutés 5mL d'eau et 66mL de méthanol. Enfin, à température ambiante, 2,1eq de triéthylamine sont ajoutés ce qui amorce la précipitation. Le milieu réactionnel est alors laissé sous agitation pendant 30minutes. Le précipité est ensuite filtré puis remis en suspension dans environ 50mL d'eau distillée. Le mélange est porté sous agitation et chauffé à 65°C pendant 20min. Le précipité est à nouveau filtré, lavé avec 13mL de méthanol puis 13mL d'éther diéthylique. Le produit obtenu est séché sous vide à 25°C pendant une nuit puis recristallisé en ajoutant 50mL d'une solution isopropanol : eau / 1:1 et en chauffant au reflux de l'isopropanol jusqu'à dissolution complète. Le mélange est ensuite ramené à température ambiante et le produit cristallise. Le précipité est alors lavé avec 5mL du mélange isopropanol : eau / 1 : 1 puis avec 15mL de méthanol et enfin 15mL d'éther diéthylique. Le produit est isolé sous forme de cristaux blancs et séché sous vide à 20°C pendant une nuit.
Rendement : 40% 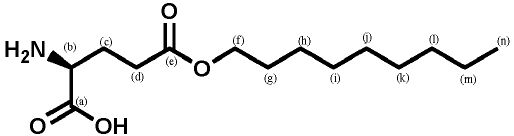
RMN (¹H, MeOD) : δ (ppm) : 0,9 (t, 3H, CH₃₍ₙ₎) ; 1,3 (m, 12H, CH₂₍ₘ₋ₕ₎) ; 1,7 (quint, 2H, CH_{2(g)}) ; 2,1 (m, 2H, CH_{2(c)}) ; 2,5 (t, 2H, CH_{2(d)}) ; 3,6 (t, 1H, CH_{(b)}) ; 4,1 (t, 2H, CH_{2(f)}).
RMN (¹³C, MeOD) : δ (ppm) : 14,5 (CH₃₍ₙ₎) ; 23,7 (CH₂₍ₘ₎) ; 27,1 (CH_{2(c)}) ; 27,5 (CH₂₍ₕ₎) ; 29,8 (CH_{2(g)}) ; 30,4 (CH₂₍ₖ₎) ; 30,5 (CH₂₍ᵢ₎) ; 30,7 (CH₂₍ⱼ₎) ; 31,1(CH_{2(d)}) ; 33,1 (CH₂₍ₗ₎) ; 55,5 (CH_{(b)}) ; 66,0 (CH_{2(f)}) ; 174,5 (C₍ₑ₎); 177,9 (C₍ₐ₎).
Anal Cale pour C₁₄H₂₇N₂O₄ (273,37) : théo : C 61,51 ; H 9,96 ; N 5,12 ; Exp : C 61,80 ; H 9,96 ; N 4,72
SM : ESI+ : [M+H]⁺ : 274,2 (100%)

### Exemple 4

### 5-(n-dodecyl)-L-glutamate ou acide 2-amino-5-(dodecyloxy)-5-oxopentanoïque

L'acide L-glutamique (5g, 1eq) est mis en suspension dans un mélange de *tert-*butanol (34 ml, 10,5eq) et de 1-dodécanol (22.9g, 3,6eq). Le milieu réactionnel est chauffé à 40 °C. H₂SO₄ 95% (2.9 ml) est additionné goutte à goutte. La suspension est chauffée à 65 °C. Après une heure et demie, on obtient une solution homogène. Le mélange est agité une heure supplémentaire à 65 °C et le chauffage est coupé. Après retour à température ambiante, de la triéthylamine (2.7 ml) est ajoutée goutte à goutte en 1 minute. Ensuite, de l'eau (8 ml) et de l'éthanol (119 ml) sont ajoutés, suivis par de la triéthylamine (10.2 ml). Après 30 minutes d'agitation, le précipité blanc est filtré et séché. Le solide blanc pâteux ainsi obtenu est trituré dans de l'eau (91 ml) à 65 °C. Le solide est filtré, lavé par du méthanol (23 ml) puis de l'éther diéthylique (23 ml) et séché sous vide. La poudre blanche obtenue (5.72g) est alors triturée dans un mélange isopropanol : eau / 2 : 1 (160ml) à 85 °C. Le mélange est refroidi à température ambiante et le produit attendu est filtré sur frité, rincé par un mélange isopropanol : eau / 2 : 1 (50 ml) puis du méthanol (50 ml) et de l'éther diéthylique (50 ml). Le produit est séché sous vide pendant la nuit puis trituré dans de l'eau (100 ml) et enfin cristallisé une deuxième fois dans un mélange isopropanol : eau / 2 :: 1 (150ml).

Le produit est enfin isolé sous forme de cristaux blancs et séché sous vide à 20°C pendant une nuit.
Rendement 43% 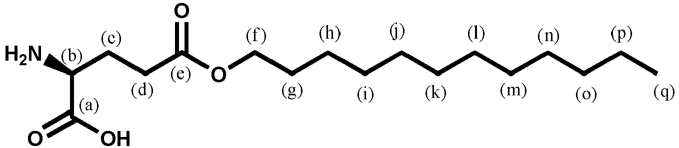
PF : 189.9°C
RMN (¹H, CF₃COOD, 300 MHz) : δ (ppm) : 0,9 (t, 3H, CH_{3(q)}) ; 1,3 (m, 18H, CH₂₍ₚ₋ₕ₎) ; 1,8 (m, 2H, CH_{2(g)}) ; 2,5 (m, 2H, CH_{2(c)}) ; 2,95 (t, 2H, CH_{2(d)});4,3 (t, 2H, CH_{2(f)}) ; 4,58 (m, 1H, CH_{(b)}).
RMN (¹³C, CF₃COOD, 75 MHz) : δ (ppm) : 12,3 (CH_{3(q)}) ; 21,9 (CH₂₍ₚ₎) ; 24,2 (CH_{2(c)}) ; 25,01 (CH₂₍ₕ₎) ; 27,5 (CH_{2(g)}) ; 28,5-29,01 (CH₂₍ᵢ₋ₙ₎) ; 30,01 (CH_{2(d)}) ; 31,3 (CH_{2(O)}) ; 53,4 (CH_{(b)}) ; 67,6 (CH_{2(f)}) ; 172,5 (C₍ₑ₎) ; 176,6 (C₍ₐ₎).
Anal Cale pour C₁₇H₃₃NO₄ (315,24) : théo : C 64,73 ; H 10,54 ; N 4,44 ; Exp : C 64,57 ; H 10,69 ; N 4,10
SM : ESI+ : [M+H]⁺ : 316,2 ; ESI- : [M-H]⁻ : 314,2

### Exemple 5

### 5-(n-tetradecyl)-L-glutamate ou acidee 2-amino-5-oxo-5(tetradecyl)pentanoïque

Ce composé isolé sous forme de solide blanc a été obtenu avec un rendement de 51 % suivant le protocole utilisé pour la préparation du L-Glutamic acid 5 dodécyl ester avec 4 g d'acide glutamique et 23.3g de tétradécanol. 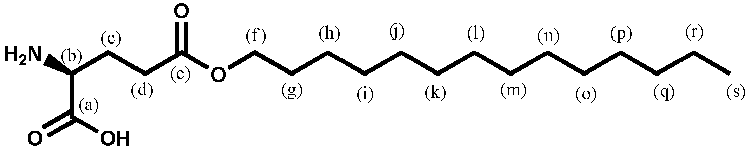
PF : 184.8°C
RMN (¹H, CF₃COOD, 300 MHz) : δ (ppm) : 0,9 (t, 3H, CH₃₍ₛ₎) ; 1,3-1,4 (m, 22H, CH₂₍ᵣ₋ₕ₎) ; 1,8 (m, 2H, CH_{2(g)}) ; 2,5 (m, 2H, CH_{2(c)}) ; 2,95 (t, 2H, CH_{2(d)});4,3 (t, 2H, CH_{2(f)}) ; 4,58 (m, 1H, CH_{(b)}).
RMN (¹³C, CF3COOD, 75 MHz) : δ (ppm) : 12,2 (CH₃₍ₛ₎) ; 21,9 (CH₂₍ᵣ₎) ; 24,2 (CH_{2(c)}) ; 25,01 (CH₂₍ₕ₎) ; 27,5 (CH_{2(g)}) ; 28,5-29,07 (CH₂₍ᵢ₋ₚ₎) ; 30,00 (CH_{2(d)}) ; 31,4 (CH_{2(q)}) ; 53,4 (CH_{(b)}) ; 67,6 (CH_{2(f)}) ; 172,5 (C₍ₑ₎) ; 176,6 (C₍ₐ₎).
Anal Cale pour C₁₉H₃₇NO₄ (343,27) : théo : C 66,43 ; H 10,86 ; N 4,08 ; Exp : C 65,66 ; H 10,58 ; N 3,94
SM : ESI+ : [M+H]⁺ : 344,2 ; ESI- : [M-H]⁻ : 342,2

### Exemple 6

### 5-(n-hexadécyl)-L-glutamate ou acide 2-amino-5-(hexadécyloxy)-5-oxopentanoïque

Ce composé isolé sous forme de solide blanc a été obtenu avec un rendement de 69% suivant le protocole utilisé pour la préparation de du 5-(n-dodécyl)-L-glutamate avec 5g d'acide glutamique et 33g de hexadécanol. 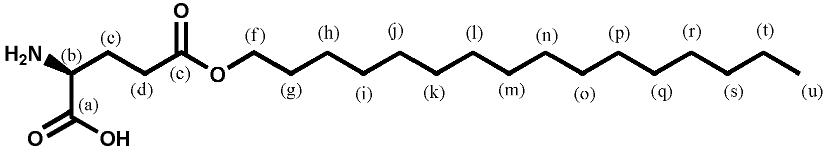
PF : 179.3°C
RMN (¹H, CF₃COOD, 300 MHz) : δ (ppm) : 0,8 (t, 3H, CH₃₍ᵤ₎) ; 1,23 (m, 26H, CH₂₍ₜ₋ₕ₎) ; 1,65 (m, 2H, CH_{2(g)}) ; 2,4 (m, 2H, CH_{2(c)}) ; 2,8 (t, 2H, CH_{2(d)}); 4,16 (t, 2H, CH_{2(f)}) ; 4,43 (m, 1H, CH_{(b)}).
RMN (¹³C, CF₃COOD, 75 MHz) : δ (ppm) : 12,2 (CH₃₍ᵤ₎) ; 21,9 (CH₂₍ₜ₎) ; 24,1 (CH_{2(c)}) ; 24,9 (CH₂₍ₕ₎) ; 27,4 (CH_{2(g)}) ; 28,4-28,9 (CH₂₍ᵢ₋ᵣ₎) ; 29,8 (CH_{2(d)}) ; 31,3 (CH₂₍ₛ₎) ; 53,2 (CH_{(b)}) ; 67,4 (CH_{2(f)}) ; 172,3 (C₍ₑ₎) ; 176,5 (C₍ₐ₎).
Anal Cale pour C₂₁H₄₁NO₄ (371,55) : théo : C 67,88 ; H 11,12 ; N 3,77 ; Exp : C 67,60 ; H 10,93 ; N 3,77
SM : ESI+ : [M+H]⁺ : 372,3 ; ESI- : [M-H]⁻ : 370,2

### Exemple 7

### 5-(n-octadécyl)-L-glutamate ou acide 2-amino-5-(octadécyloxy)-5-oxopentanoïque

Ce composé est isolé sous forme de solide blanc avec un rendement de 45% suivant le protocole utilisé pour la préparation du 5-(n-dodécyl)-L-glutamate ester avec 2.5g d'acide glutamique et 18.1g d'octadécanol. 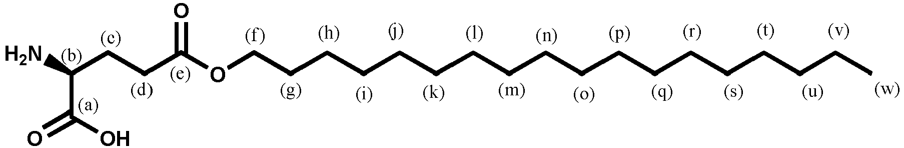
PF : 180.5°C
RMN (¹H, CF₃COOD, 300 MHz) : δ (ppm) : 0,8 (t, 3H, CH_{3(w)}) ; 1,3 (m, 30H, CH₂₍ᵥ₋ₕ₎) ; 1,7 (m, 2H, CH_{2(g)}) ; 2,5 (m, 2H, CH_{2(c)}) ; 2,9 (t, 2H, CH_{2(d)}); 4,2 (t, 2H, CH_{2(f)}) ; 4,5 (m, 1H, CH_{(b)}).
RMN (¹³C, CF₃COOD, 75 MHz) : δ (ppm) : 12,2 (CH_{3(w)}) ; 21,9 (CH₂₍ᵥ₎) ; 24,1 (CH_{2(c)}) ; 24,9 (CH₂₍ₕ₎) ; 27,5 (CH_{2(g)}) ; 28,5-29,01 (CH₂₍ᵢ₋ₜ₎) ; 29,9 (CH_{2(d)}) ; 31,3 (CH₂₍ᵤ₎) ; 53,3 (CH_{(b)}) ; 67,5 (CH_{2(f)}) ; 172,4 (C₍ₑ₎) ; 176,6 (C₍ₐ₎).
Anal Cale pour C₂₃H₄₅NO₄ (399,61) : théo : C 69,13 ; H 11,35 ; N 3,51 ; Exp : C 68,69 ; H 11,36 ; N 3,38
SM : ESI+ : [M+H]⁺ : 400,3 ; ESI- : [M-H]⁻ : 398,3

### Exemple 8

### 5-(3,7-diméthyloctyl)-L-glutamate ou acide 2-amino-5(3,7-diméthyloctyloxy)-5-oxopentanoïque

Ce composé est isolé sous forme de solide blanc avec un rendement de 51 % suivant le protocole utilisé pour la préparation du 5-(n-dodécyl)-L-glutamate ester avec 3g d'acide glutamique et 15.6 ml de 3,7-dimethyl-1-octanol. 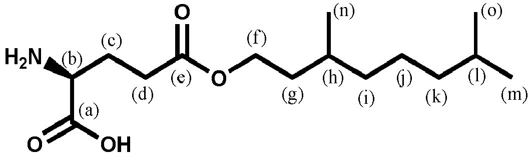
PF : 150.8°C
RMN (¹H, CF₃COOD, 300 MHz) : δ (ppm) : 0,95 (m, 6H, CH₃₍ₘ₊ₒ₎) ; 0,98 (m, 3H, CH₃₍ₙ₎) ; 1,3-1,4 (m, 6H, CH₂₍ᵢ₋ₖ₎) ; 1,7 (m, 3H, CH_{2(g)}+ CH₍ₗ₎) ; 1,9 (m, 1H, CH₍ₕ₎); 2,5 (m, 2H, CH_{2(c)}) ; 2,9 (m, 2H, CH_{2(d)}); 4,4 (t, 2H, CH_{2(f)}) ; 4,6 (m, 1H, CH_{(b)}).
RMN (¹³C, CF₃COOD, 75 MHz) : δ (ppm) : 17,3 (CH₃₍ₙ₎) ; 20,5 (CH₃₍ₘ₎+ CH₃₍ₒ₎) ; 23,7 (CH_{2(c)}+ CH₂₍ⱼ₎); 27,06 (CH₍ₗ₎) ; 29,07 (CH₍ₕ₎); 29,7 (CH_{2(d)}) ; 34,15 (CH_{2(g)}) ; 36,16 (CH₂₍ᵢ₎) ; 38,3 (CH₂₍ₖ₎) ; 53,05 CH_{(b)}) ; 65,8 (CH_{2(f)}) ; 172,2 (C₍ₑ₎) ; 176,3 (C₍ₐ₎).
Anal Cale pour C₁₅H₂₉NO₄ (287,4) : théo : C 62,69 ; H 10,17 ; N 4,87 ; Exp : C 62,42 ; H 10,26 ; N 4,64
SM : ESI+ : [M+H]⁺ : 288,3 ; ESI- : [M-H]⁻ : 286,3

### Procédure 2

Synthèse utilisant l'acide (S)-5-(benzyloxy)-4-(benzyloxycarbonylamino)-5-oxopentanoïque comme produit de départ. Les fonctions protégées par des benzyles sont alors déprotégées par hydrogénation catalytique.

### Mise en évidence de l'activité immuno-régulatrice sur IL2

Le test précédemment décrit montre pour ce composé de l'exemple 8 est doté d'une activité significative de - 29 % à 300 µM sur la sécrétion d'IL2.

### Exemple 9

### 5-(n-nonan-2-yl)-L-glutamate ou acide 2-amino-5(nonan-2-yloxy)-5-oxopentanoïque

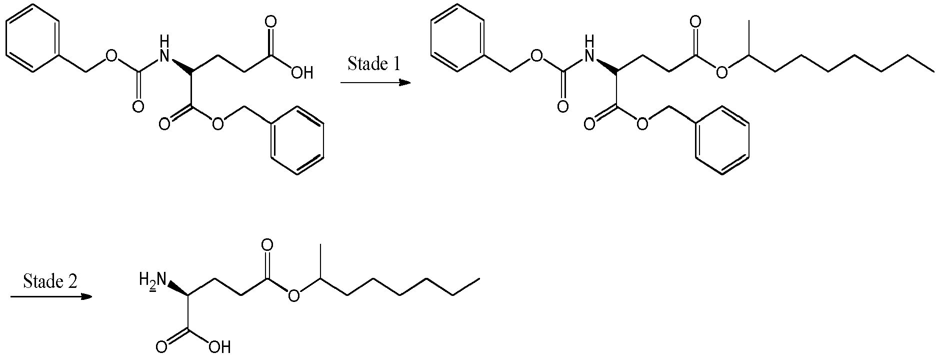

Equipement: monocol 250 ml muni d'une agitation magnétique et placé sous balayage d'azote

L'acide alpha-(n-benzyl)-L-glutamate-N-alpha-benzylcarbonyle (5g, 1eq), le 2-nonanol (2.92g, 1,5eq), l'EDCI (2.58g, 1eq), l'HOBT (1.82g, 1eq) et le DMAP (1.65g, 1eq) sont mis en solution dans du dichlorométhane (100 ml). Le milieu réactionnel est agité à température ambiante pour la nuit. Le milieu est lavé successivement par une solution d'acide chlorhydrique 0.1N, une solution d'hydrogénocarbonate de sodium saturée, puis une solution de chlorure de sodium saturée. La phase organique est séchée sur sulfate de magnésium, filtrée et concentrée pour conduire à une huile jaune pâle qui est alors purifiée par chromatographie sur gel de silice (gradient heptane : acétate d'éthyle / 100 : 0 à 50 : 50) pour conduire à une huile jaune (2.65g, rendement 40%) qui sera réengagée dans le stade 2.

Le montage est mis sous atmosphère inerte (azote). L'huile jaune est mise en solution dans le méthanol (132 ml) et le Palladium sur charbon (10%, 566 mg) est additionné. Le milieu est hydrogéné à température ambiante pour la nuit puis filtré sur célite. Le filtrat est concentré à sec pour conduire à un solide blanc qui sera trituré dans du méthanol (10 ml) à 0 °C pendant une heure. Le solide est alors filtré sur frité, rincé par du méthanol glacé et séché pour conduire à un solide blanc (1g, rendement 69%). 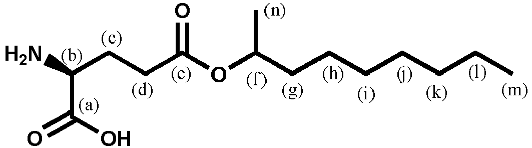
RMN (¹H, CF₃COOD, 300 MHz) : δ (ppm) : 0,9 (t, 3H, CH₃₍ₘ₎) ; 1,3 (m, 13H, CH₃₍ₙ₎ + CH₂₍ₕ₋ₗ₎) ; 1,7 (m, 2H, CH_{2(g)}) ; 2,5 (m, 2H, CH_{2(c)}) ; 2,9 (t, 2H, CH_{2(d)}); 4,5 (m, 1H, CH_{(f)}) ; 5,1 (m, 1H, CH_{(b)}).
RMN (¹³C, CF₃COOD, 75 MHz) : δ (ppm) : 12,3 (CH₃₍ₘ₎) ; 17,6 (CH₂₍ₙ₎) ; 21,7 (CH₂₍ₗ₎) ; 24,2 (CH_{2(c)}) ; 24,6 (CH₂₍ₕ₎) ; 28,4-28,5 (CH₂₍ᵢ₋ⱼ₎) ; 30,2 (CH_{2(d)}) ; 30,3 (CH₂₍ₖ₎) ; 34,9 (CH_{2(g)}) ; 53,3 (CH_{(b)}) ; 75,8 (CH_{2(f)}) ; 172,5 (C₍ₑ₎) ; 176,1 (C₍ₐ₎).
Anal Cale pour C₁₄H₂₇NO₄ (273,37) : théo : C 61,51 ; H 9,96 ; N 5,12 ; Exp : C 61,63 ; H 9,67 ; N 5,07
SM : ESI+ : [M+H]⁺ : 274,3 ; ESI- : [M-H]⁻ : 272,2

### Exemple 10

### 5-(n-nonan-5-yl)-L-glutamate ou acide 2-amino-5(nonan-5-yloxy)-5-oxopentanoïque

Ce composé est obtenu selon les mêmes conditions opératoires que celles utilisées pour l'isolement du composé 5(n-nonan-2-yl)-L-glutamate. Seule la trituration dans le méthanol au dernier stade n'a pas été mise en oeuvre à cause de la partielle solubilité de ce produit dans ce solvant.
*Stade 1 :* 1,98g d'une huile jaune qui cristallise dans le temps, rendement 60%
*Stade 2 :* 300 mg, rendement 68% 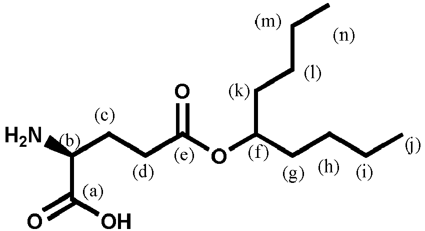
RMN (¹H, CF₃COOD, 300 MHz) : δ (ppm) : 0,9 (m, 6H, CH_{3(n) +} CH₃₍ⱼ₎) ; 1,3 (m, 8H, CH₂₍ₘ₊ₗ₊ₕ₊ᵢ₎) ; 1,4 (m, 4H, CH_{2(k+g)}) ; 2,1 (m, 2H, CH_{2(c)}) ; 2,5 (m, 2H, CH_{2(d)}); 3,5 (m, 1H, CH _{(f)}) ; 4,8 (m, 1H, CH_{(b)}).
RMN (¹³C, CF₃COOD, 75 MHz) : δ (ppm) : 17,5 (CH₃₍ⱼ₎₊ CH₃₍ₙ₎) ; 24,04 (CH₂₍ₗ₊ₘ₊ₕ₊ᵢ₎) ; 28,8 (CH_{2(c)}) ; 31,5 (CH_{2(d)}) ; 35,3 (CH_{2(g+k)}) ; 55,7 (CH_{(b)}) ; 76,4 (CH_{2(f)}) ; 173,4 (C₍ₑ₎) ; 174,6 (C₍ₐ₎).
Anal Cale pour C₁₄H₂₇NO₄ (273,37) : théo : C 61,51 ; H 9,96 ; N 5,12 ; Exp : C 61,17 ; H 9,91 ; N 5,03
SM : ESI+ : [M+H]⁺ : 274,3 ; ESI- : [M-H]⁻ : 272,2

### Exemple 11

### 5-(2-hexyldécyl)-L-glutamate ou acide 2-amino-5(2-hexyldécyl oxy)-5-oxopentanoïque

Ce composé est obtenu selon les mêmes conditions opératoires que celles utilisées pour l'isolement du composé 5-(nonan-2-yl)-L-glutamate. Le faible rendement du dernier stade s'explique par une partielle solubilité du produit dans le méthanol.
*Stade 1 :* 3.93g d'un solide blanc, rendement 57%
*Stade 2 :* 440 mg d'un solide blanc, rendement 18% 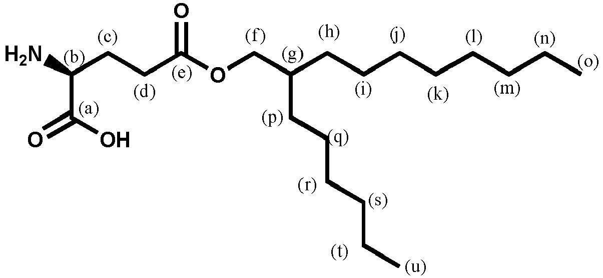
RMN (¹H, CF₃COOD, 300 MHz) : δ (ppm) : 0,9 (m, 6H, CH_{3(o) +} CH₃₍ᵤ₎) ; 1,3 (m, 24H, CH_{2(h+i+j+k+l+m+n+p+q+r+s+t)}) ; 1,7 (m, 1H, CH_{g}) ; 2,5 (m, 2H, CH_{2(c)}) ; 2,9 (m, 2H, CH_{2(d)}); 4,2 (m, 2H, CH_{2(f)}) ; 4,5 (m, 1H, CH_{(b)}).
RMN (¹³C, CF₃COOD, 75 MHz) : δ (ppm) : 12,1 (CH_{3(O)+}CH₃₍ᵤ₎) ; 21,7 (CH₂₍ₙ₊ₜ₎) ; 24,1 (CH_{2(c)}) ; 25,8 (CH_{2(i+q)}) ; 28,6-31,2 (CH_{2(d+j+k+r+h+p+m+l+s)}) ; 36,7 (CH_{g}) ; 53,3 (CH_{(b)}) ; 70,3 (CH_{2(f)}) ; 172,4 (C₍ₑ₎) ; 176,6 (C₍ₐ₎).
Anal Cale pour C₂₁H₄₁NO₄ (371,3) : théo : C 67,88 ; H 11,12 ; N 3,77 ; Exp : C 67,78 ; H 11,08 ; N 3,76
SM : ESI+ : [M+H]⁺ : 372,3 ; ESI- : [M-H]⁻ : 370,3

### Exemple 12

### 5-(2-ethylhexyl)-L-glutamate ou acide 2-amino-5(2-ethylhexyl oxy)-5-oxopentanoïque

Ce composé est obtenu selon les mêmes conditions opératoires que celles utilisées pour l'isolement du composé 5-(nonan-2-yl)-L-glutamate. Une variante intervient au niveau du traitement du stade 2. En effet, après filtration sur célite, le filtrat est concentré jusqu'à environ 50 ml. De l'éther diisopropylique (200 ml) est ajouté et le mélange formé est placé à -18 °C pendant une nuit. La suspension ainsi obtenue est filtrée et le solide blanc est séché sous vide.
*Stade 1 :* 4.22g d'une huile incolore, rendement 65%
*Stade 2 :* 1,5g d'un solide blanc, rendement 70% 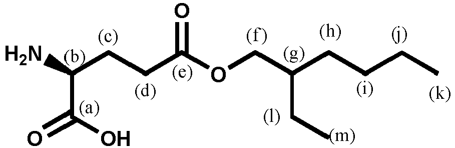
PF : 157.9°C
RMN (¹H, CF₃COOD, 300 MHz) : δ (ppm) : 0,9 (m, 6H, CH_{3(k) +} CH₃₍ₘ₎) ; 1,4 (m, 8H, CH₂₍ₕ₊ᵢ₊ⱼ₊ₗ₎) ; 1,7 (m, 1H, CH_{g}) ; 2,6 (m, 2H, CH_{2(c)}) ; 2,9 (m, 2H, CH_{2(d)}); 4,2 (m, 2H, CH_{2(f)}) ; 4,5 (m, 1H, CH_{(b)}).
RMN (¹³C, CF₃COOD, 75 MHz) : δ (ppm) : 8,9 (CH₃₍ₘ₎) ; 11,9 (CH₃₍ₖ₎) ; 22,0 (CH₂₍ⱼ₎) ; 22,8 (CH₂₍ₗ₎) ; 24,2 (CH_{2(c)}) ; 28,2 (CH₂₍ᵢ₎), 29,5 (CH₂₍ₕ₎); 29,9 (CH_{2(d)}); 38,3 (CH_{g}) ; 53,3 (CH_{(b)}) ; 69,9 (CH_{2(f)}) ; 172,5 (C₍ₑ₎) ; 176,6 (C₍ₐ₎).
Anal Calc pour C₁₃H₂₅NO₄ (259,2) : théo : C 60,21 ; H 9,72 ; N 5,40 ; Exp : C 60,08 ; H 9,59 ; N 5,45
SM : ESI+ : [M+H]⁺ : 260,2 ; ESI- : [M-H]⁻ : 258,1

### Evaluation sur modèle de kératinocytes du composé selon l'exemple 12:

Mise en évidence de l'activité anti-inflammatoire sur des kératinocytes exprimant un phénotype de dermatite atopique (DA) : Etude des cytokines et chemokines surexprimées dans la pathologie de la dermatite atopique.

Les kératinocytes primaires (NHEK, Normal Human Epidermal Keratinocytes) distribués en plaque 24 puits sont préincubés pendant 1 heure avec les principes actifs puis stimulés pendant 24 heures avec un cocktail de 4 agonistes permettant de créer in vitro l'environnement physiopathologique de la dermatite atopique : 100 ng/ml IL4 + 100 ng/ml IL13 + 1 µg/ml Poly I:C + 5 µg/ml Pam3CSK4 : ce modèle permet d'induire l'inflammation médiée par les cytokines Th2 surexprimées dans la DA (IL4, IL13) et par les composantes bactérienne (mimée par Pam3CSK4, ligand TLR-2) et virale (mimée par Poly I:C, ligand TLR-3) de la DA. L'analyse de l'expression génique des marqueurs de la DA est réalisée en PCR-Array 48 gènes et répétée sur 3 donneurs différents de NHEK.

La modulation de la réponse inflammatoire observée est illustrée par le tableau annexé sur lequel :

Pour l'ensemble des marqueurs, le RQ (Relative Quantity) inférieur à 0.5, à 5 h ou 24 h, indique une inhibition significative :
→ inhibition du marqueur TSLP impliqué dans la polarisation lymphocytaire Th2 et spécifique de la DA ;
→ inhibition des cytokines inflammatoires IL1A, IL8, CSF2, IFNB1, VEGF ;
→ inhibition des chemokines CCL, CXCL, impliquées dans le recrutement, l'infiltration cutanée, et l'activation des cellules inflammatoires (leucocytes, lymphocytes, lymphocytes Th1 et Th2, monocytes).

Les résultats obtenus de la mise en évidence de l'activité anti-inflammatoire de l'exemple 12 vis-à-vis de l'expression génique des marqueurs inflammatoires de la DA, cytokines et chemokines sont donnés dans le tableau ci-après:

| Kératinocytes NHEK : marqueurs de l'inflammation induits par 100 ng/ml IL4 + 100 ng/ml IL13 + 1 µg/ml Poly I :C + 5 µg/ml Pam3CSK4 | | Activité à 24h du composé selon ex 12 | |
|---|---|---|---|
| | | 100 µM | 300 µM |
| *Cytokines* | | | |
| Interleukin 1, alpha | IL1A | 0,53 | 0,53 |
| Interleukin 8 | IL8 | 0,29 | 0,18 |
| Thymic stromal lymphopoietin | TSLP | 0,20 | 0,54 |
| GMCSF, granulocyte-macrophage colony stimulating factor 2 | CSF2 | 1,55 | 0,33 |
| Interferon beta 1 | IFNB1 | 0,19 | 0,26 |
| Vascular endothelial growth factor C | VEGFC | 0,20 | 0,50 |

| *Chimiokines* | | | |
|---|---|---|---|
| MCP-1, Monocyte chemoattractant protein 1 | CCL2 | 0,48 | *nd* |
| MIP-1α, Macrophage inflammatory protein 1-alpha | CCL3 | 0,51 | 0,06 |
| MIP-1β, Macrophage inflammatory protein 1-beta | CCL4 | 0,14 | 0,02 |
| RANTES, Regulated on Activation, Normal T cell Expressed and Secreted | CCL5 | 0,42 | 0,20 |
| MIP-3α, macrophage inflammatory protein 3-alpha | CCL20 | 0,13 | 0,13 |
| GRO-α, Melanoma growth stimulating activity, alpha | CXCL1 | 0,33 | 0,36 |
| MIP-2α, macrophage inflammatory protein 2-alpha | CXCL2 | 0,06 | 0,32 |
| CXCL9, Gamma-interferon-induced monokine | CXCL9 | 0,20 | 0,29 |
| IP-10, 10 kDa Interferon gamma-induced protein | CXCL10 | 0,18 | 0,06 |
| I-TAC, Interferon-inducible T-cell alpha chemoattractant | CXCL11 | 0,14 | 0,15 |

| |
|---|
| Activités exprimées en RQ (Relative Quantity) par rapport au Témoin stimulé 100 ng/ml IL4+100 ng/ml IL13 + 1 µg/ml Poly I :C + 5 µg/ml Pam3CSK4 / RQ<0.5 = inhibition du marqueur de l'inflammation |

### Exemple de formulation d'un principe actif selon la présente invention

La formulation a été développée spécifiquement pour ce type de composés de formule I, en particulier dans lesquels le radical R est un alkyle linéaire ou ramifié de 5 à 25 atomes de carbone, présentant généralement une très faible hydro solubilité.

La formulation suivante correspond au composé de l'exemple 12, mais peut parfaitement être généralisée aux autres composés de formule I à substitution alkylique en C₅ à C₂₅ :

| | |
|---|---|
| Composé en ex 12 | 1.0 |
| Cyclométhicone | 3.5 |
| Emollient MYRITOL 318 | 3.5 |
| Emollient CETIOL HE | 3.5 |
| Gélifiant SEPIPLUS400 | 3.0 |
| Propylène glycol | 9.0 |
| Phénoxyéthanol | 0.2 |
| Eau purifiée | qsp 100 ml |

La formulation ci-dessus a été préparée comme suit :
Phase A : Chauffer le mélange eau et phénoxyéthanol à 75°C. Maintenir la chauffe et ajouter le gélifiant sous forte agitation.
Phase B : Mélanger les émollients et le silicone et chauffer ce mélange à 75°C.
Phase C : Mélanger le propylène glycol et le composé selon l'exemple 12 : chauffer ce mélange à 80°C pendant 30 minutes.
Emulsionner B dans A puis ajouter la phase C.

## Revendications

1. Composés dérivés du L-glutamate de formule générale I suivante : 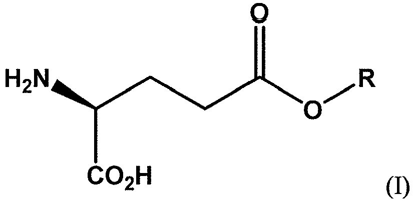 dans laquelle le radical R représenteun groupe alkyle linéaire ou ramifié en C₅ à C₂₅ pour leur utilisation topique en tant que principe actif dermatologique destiné au traitement et/ou la prévention des dermatoses inflammatoires.

2. Composés pour utilisation selon la revendication 1 **caractérisés en ce que** le radical R est un groupe alkyle linéaire ou ramifié en C5 à C14.

3. Composé de formule générale (I) pour utilisation selon la revendication 1, **caractérisé en ce qu'**il est choisi parmi l'un des composés suivants :
- 5-(n-pentyl)-L-glutamate ou acide 2-amino-5-oxo-5-(pentyloxy)pentanoïque
- 5-(n-hexyl)-L-glutamate ou acide 2-amino-5-(hexyloxy)-5-oxopentanoïque
- 5-(n-nonyl)-L-glutamate ou acide 2-amino-5-(nonyloxy)-5-oxopentanoïque
- 5-(n-dodécyl)-L-glutamate ou acide 2-amino-5-(dodécyloxy)-5-oxopentanoïque
- 5-(n-tétradécyl)-L-glutamate ou acide 2-amino-5-oxo-5(tétradécyl)pentanoïque
- 5-(n-hexadecyl)-L-glutamate ou acide 2-amino-5-(hexadecyloxy)-5-oxopentanoïque
- 5-(n-octadécyl)-L-glutamate ou acide 2-amino-5-(octadécyloxy)-5-oxopentanoïque
- 5-(3,7-diméthyloctyl)-L-glutamate ou acide 2-amino-5(3,7-dimethyloctyloxy)-5-oxopentanoïque
- 5-(nonan-2-yl)-L-glutamate ou acide 2-amino-5(nonan-2-yloxy)-5-oxopentanoïque
- 5-(nonan-5-yl)-L-glutamate ou acide 2-amino-5(nonan-5-yloxy)-5-oxopentanoïque
- 5-(2-hexyldecyl)-L-glutamate ou acide 2-amino-5(2-hexyldecyl oxy)-5-oxopentanoïque
- 5-(2-éthylhexyl)-L-glutamate ou acide 2-amino-5(2-éthylhexyl oxy)-5-oxopentanoïque.

4. Composé de formule générale (I) pour utilisation selon l'une des revendications 1 à 3, **caractérisé en ce que** la dermatose inflammatoire consiste en une dermatite atopique, un eczéma de contact, un acné, une dermatite séborrhéique, une rosacée ou un psoriasis.

5. Composition topique **caractérisée en ce qu'**elle comprend à titre de principe actif un composé de formule I tel que défini à l'une des revendications 1 à 3, en association avec au moins un excipient pharmaceutiquement acceptable pour son utilisation topique en tant que médicament destiné au traitement et/ou à la prévention de dermatoses inflammatoires.

6. Composés dérivés de l'acide L-glutamique de formule générale (I) **caractérisé en ce qu'**il est choisi parmi :
- 5-(3,7-diméthyloctyl)-L-glutamate ou acide 2-amino-5(3,7-diméthyloctyloxy)-5-oxopentanoïque
- 5-(nonan-2-yl)-L-glutamate ou acide 2-amino-5(nonan-2-yloxy)-5-oxopentanoïque
- 5-(nonan-5-yl)-L-glutamate ou acide 2-amino-5(nonan-5-yloxy)-5-oxopentanoïque
- 5-(2-hexyldécyl)-L-glutamate ou acide 2-amino-5(2-hexyldécyl oxy)-5-oxopentanoïque
- 5-(2-éthylhexyl)-L-glutamate ou acide 2-amino-5(2-éthylhexyl oxy)-5-oxopentanoïque.

7. Procédé de préparation des composés de formule I définis à la revendication 6, **caractérisé en ce que** l'on fait réagir l'acide alpha-(n-benzyl)-L-glutamate-N-alpha-benzylcarbonyle avec un alcool de formule R-OH où R à la signification correspondante donnée à la revendication 1 et **en ce que** le produit obtenu est déprotégé par hydrogénation catalytique.

## Patentansprüche

1. L-Glutamat-Derivatverbindungen mit der folgenden allgemeinen Formel I: 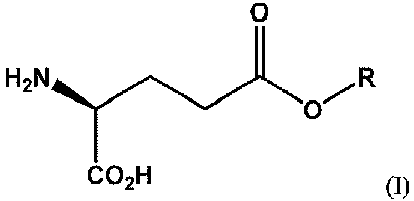 wobei das Radikal R eine lineare oder verzweigte C₅-C₂₅-Alkylgruppe darstellt, für ihre topische Verwendung als dermatologischer Wirkstoff, der zur Behandlung und/oder Prävention entzündlicher Hautkrankheiten bestimmt ist.

2. Verbindungen zur Verwendung nach Anspruch 1, **dadurch gekennzeichnet, dass** das Radikal R eine lineare oder verzweigte C₅-C₁₄-Alkylgruppe ist.

3. Verbindung mit der allgemeinen Formel (I) zur Verwendung nach Anspruch 1, **dadurch gekennzeichnet, dass** sie unter einer der folgenden Verbindungen ausgewählt ist:
- 5-(n-Pentyl)-L-Glutamat oder 2-Amino-5-oxo-5-(pentyloxy)pentansäure
- 5-(n-Hexyl)-L-Glutamat oder 2-Amino-5-(hexyloxy)-5-Oxopentansäure
- 5-(n-Nonyl)-L-Glutamat oder 2-Amino-5-(nonyloxy)-5-Oxopentansäure
- 5-(n-Dodecyl)-L-Glutamat oder 2-Amino-5-(dodecyloxy)-5-Oxopentansäure
- 5-(n-Tetradecyl)-L-Glutamat oder 2-Amino-5-oxo-5 (Tetradecyl)pentansäure
- 5-(n-Hexadecyl)-L-Glutamat oder 2-Amino-5-(hexadecyloxy)-5-Oxopentansäure
- 5-(n-Octadecyl)-L-Glutamat oder 2-Amino-5-(octadecyloxy)-5-Oxopentansäure
- 5-(3,7-Dimethyloctyl)-L-Glutamat oder 2-Amino-5 (3,7-dimethyloctyloxy)-5-Oxopentansäure
- 5-(Nonan-2-yl)-L-Glutamat oder 2-Amino-5(nonan-2-yloxy)-5-Oxopentansäure
- 5-(Nonan-5-yl)-L-Glutamat oder 2-Amino-5(nonan-5-yloxy)-5-Oxopentansäure
- 5-(2-Hexyldecyl)-L-Glutamat oder 2-Amino-5(2-hexyldecyloxy)-5-Oxopentansäure
- 5-(2-Ethylhexyl)-L-Glutamat oder 2-Amino-5(2-ethylhexyloxy)-5-Oxopentansäure.

4. Verbindung mit der allgemeinen Formel (I) zur Verwendung nach einem der Ansprüche 1 bis 3, **dadurch gekennzeichnet, dass** die entzündliche Hautkrankheit in einer atopischen Dermatitis, einem Kontaktekzem, einer Akne, einer seborrhoischen Dermatitis, einer Rosacea oder einer Schuppenflechte besteht.

5. Topische Zusammensetzung, **dadurch gekennzeichnet, dass** sie als Wirkstoff eine Verbindung mit der Formel I nach einem der Ansprüche 1 bis 3 in Verbindung mit mindestens einem pharmazeutisch verträglichen Trägerstoff für ihre topische Verwendung als Medikament umfasst, das zur Behandlung und/oder Prävention von entzündlichen Hautkrankheiten bestimmt ist.

6. L-Glutaminsäure-Derivatverbindungen mit der allgemeinen Formel (I), **dadurch gekennzeichnet, dass** sie unter Folgendem ausgewählt sind:
- 5-(3,7-Dimethyloctyl)-L-Glutamat oder 2-Amino-5 (3,7-dimethyloctyloxy)-5-Oxopentansäure
- 5-(Nonan-2-yl)-L-Glutamat oder 2-Amino-5(nonan-2-yloxy)-5-Oxopentansäure
- 5-(Nonan-5-yl)-L-Glutamat oder 2-Amino-5(nonan-5-yloxy)-5-Oxopentansäure
- 5-(2-Hexyldecyl)-L-Glutamat oder 2-Amino-5(2-hexyldecyloxy)-5-Oxopentansäure
- 5-(2-Ethylhexyl)-L-Glutamat oder 2-Amino-5(2-ethylhexyloxy)-5-Oxopentansäure.

7. Verfahren zur Zubereitung der Verbindungen mit der allgemeinen Formel I nach Anspruch 6, **dadurch gekennzeichnet, dass** die Alpha-(n-benzyl)-L-Glutamat-N-alpha-Benzylcarbonylsäure mit einem Alkohol mit der Formel R-OH zur Reaktion gebracht wird, wobei R die entsprechenden in Anspruch 1 gegebene Bedeutung hat und dadurch, dass das erhaltene Produkt durch katalytische Hydrierung entschützt wird.

## Claims

1. Compounds derived from L-glutamate of the following general formula I: 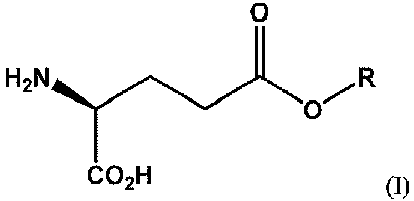 wherein the radical R represents a linear or branched C5-C25 alkyl group, for topical use as a dermatological active ingredient in the treatment and/or prevention of inflammatory dermatoses.

2. Compounds for use according to claim 1, **characterized in that** the radical R is a linear or branched C5-C14 alkyl group.

3. A compound of the general formula (I) for use according to claim 1, **characterized in that** said compound is selected from one of the following compounds:
- 5-(n-pentyl)-L-glutamate or 2-amino-5-oxo-5-(pentyloxy)pentanoic acid
- 5-(n-hexyl)-L-glutamate or 2-amino-5-(hexyloxy)-5-oxopentanoic acid
- 5-(n-nonyl)-L-glutamate or 2-amino-5-(nonyloxy)-5-oxopentanoic acid
- 5-(n-dodecyl)-L-glutamate or 2-amino-5-(dodecyloxy)-5-oxopentanoic acid
- 5-(n-tetradecyl)-L-glutamate or 2-amino-5-oxo-5(tetradecyl)pentanoic acid
- 5-(n-hexadecyl)-L-glutamate or 2-amino-5-(hexadecyloxy)-5-oxopentanoic acid
- 5-(n-octadecyl)-L-glutamate or 2-amino-5-(octadecyloxy)-5-oxopentanoic acid
- 5-(3,7-dimethyloctyl)-L-glutamate or 2-amino-5(3,7-dimethyloctyloxy)-5-oxopentanoic acid
- 5-(nonan-2-yl)-L-glutamate or 2-amino-5(nonan-2-yloxy)-5-oxopentanoic acid
- 5-(nonan-5-yl)-L-glutamate or 2-amino-5(nonan-5-yloxy)-5-oxopentanoic acid
- 5-(2-hexyldecyl)-L-glutamate or 2-amino-5(2-hexyldecyl oxy)-5-oxopentanoic acid
- 5-(2-ethylhexyl)-L-glutamate or 2-amino-5(2-ethylhexyl oxy)-5-oxopentanoic acid.

4. A compound of the general formula (I) for use according to one of claims 1 to 3, **characterized in that** the inflammatory dermatosis is atopic dermatitis, contact eczema, acne, seborrheic dermatitis, rosacea or psoriasis.

5. A topical composition **characterized in that** said composition comprises as active ingredient a compound of the formula I as defined in one of claims 1 to 3, in combination with at least one pharmaceutically acceptable excipient, for topical use as a medicinal product for the treatment and/or prevention of inflammatory dermatoses.

6. Compounds derived from L-glutamic acid of the general formula (I), **characterized in that** said compounds are selected from:
- 5-(3,7-dimethyloctyl)-L-glutamate or 2-amino-5(3,7-dimethyloctyloxy)-5-oxopentanoic acid
- 5-(nonan-2-yl)-L-glutamate or 2-amino-5(nonan-2-yloxy)-5-oxopentanoic acid
- 5-(nonan-5-yl)-L-glutamate or 2-amino-5(nonan-5-yloxy)-5-oxopentanoic acid
- 5-(2-hexyldecyl)-L-glutamate or 2-amino-5(2-hexyldecyl oxy)-5-oxopentanoic acid
- 5-(2-ethylhexyl)-L-glutamate or 2-amino-5(2-ethylhexyl oxy)-5-oxopentanoic acid.

7. A process for preparing the compounds of formula I defined in claim 6, **characterized in that** alpha-(n-benzyl)-L-glutamate-N-alpha-benzylcarbonyl acid is reacted with an alcohol having the formula R-OH where R has the corresponding meaning given in claim 1 and wherein the product obtained is deprotected by catalytic hydrogenation.
